(19) Europäisches Patentamt
European Patent Office
Office européen des brevets

(11) **EP 3 961 510 B1**

(12) **EUROPEAN PATENT SPECIFICATION**

(45) Date of publication and mention
of the grant of the patent:
**14.08.2024 Bulletin 2024/33**

(21) Application number: **21159706.7**

(22) Date of filing: **26.02.2021**

(51) International Patent Classification (IPC):
*G06N 3/04* (2023.01)    *G06N 3/08* (2023.01)
*G06N 5/00* (2023.01)    *G06N 7/00* (2023.01)
*G06N 20/10* (2019.01)    *G16H 50/70* (2018.01)

(52) Cooperative Patent Classification (CPC):
**G06F 16/2458; G06N 3/045; G06N 3/08;
G06N 5/01; G06N 7/01; G06N 20/10; G16H 50/20;
G16H 50/30; G16H 50/70**

(54) **METHOD AND SYSTEM FOR MATCHED AND BALANCED CAUSAL INFERENCE FOR MULTIPLE TREATMENTS**

VERFAHREN UND SYSTEM ZUR ANGEPASSTEN UND AUSGEWOGENEN URSÄCHLICHEN INFERENZ FÜR MEHRERE BEHANDLUNGEN

PROCÉDÉ ET SYSTÈME POUR UNE INFÉRENCE CAUSALE ADAPTÉE ET ÉQUILIBRÉE POUR PLUSIEURS TRAITEMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.04.2020 IN 202021016253**

(43) Date of publication of application:
**02.03.2022 Bulletin 2022/09**

(73) Proprietor: **Tata Consultancy Services Limited
Maharashtra (IN)**

(72) Inventors:
- **GUPTA, Garima**
  122003 Gurgaon, Haryana (IN)
- **SHARMA, Ankit**
  122003 Gurgaon - Haryana (IN)
- **PRASAD, Ranjitha**
  122003 Gurgaon - Haryana (IN)
- **CHATTERJEE, Arnab**
  122003 Gurgaon, Haryana (IN)
- **VIG, Lovekesh**
  122003 Gurgaon, Haryana (IN)
- **SHROFF, Gautam**
  122003 Gurgaon, Haryana (IN)

(74) Representative: **Goddar, Heinz J. et al
Boehmert & Boehmert
Anwaltspartnerschaft mbB
Pettenkoferstrasse 22
80336 München (DE)**

(56) References cited:
- **FREDRIK D JOHANSSON ET AL: "Learning Representations for Counterfactual Inference", ARXIV.ORG, CORNELL UNIVERSITY LIBRARY, 201 OLIN LIBRARY CORNELL UNIVERSITY ITHACA, NY 14853, 12 May 2016 (2016-05-12), XP081355168**
- **PATRICK SCHWAB ET AL: "Perfect Match: A Simple Method for Learning Representations For Counterfactual Inference With Neural Networks", ARXIV.ORG, CORNELL UNIVERSITY LIBRARY, 201 OLIN LIBRARY CORNELL UNIVERSITY ITHACA, NY 14853, 1 October 2018 (2018-10-01), XP081427889**
- **SHU YANG ET AL: "Propensity score matching and subclassification in observational studies with multi-level treatments", BIOMETRICS, JOHN WILEY, HOBOKEN, USA, vol. 72, no. 4, 17 March 2016 (2016-03-17), pages 1055 - 1065, XP071047524, ISSN: 0006-341X, DOI: 10.1111/BIOM.12505**

# EP 3 961 510 B1

**Description**

CROSS-REFERENCE TO RELATED APPLICATIONS AND PRIORITY

This patent application claims priority to India Patent Application 202021016253, filed on April 15, 2020.

TECHNICAL FIELD

The disclosure herein generally relates to the field of data mining and, more particular, to a method and system for matched and balanced causal inference for multiple treatments.

BACKGROUND

Causality is a crucial paradigm in several domains where observational data is available, such as healthcare, socioeconomic studies, advertising, etc. Primary goal of Causal Inference (CI) is to uncover cause-effect relationship between entities. An impediment to CI in observational studies is the presence of confounding, where assignment and the response to the treatment depends on context covariates, resulting in selection bias. For example, if there are two categories of entities, where a first category has 20 records and a second category has 80 records. Here, the impact of the second category may be more in CI which may further leads to selection bias.

Conventional methods obtains causal relationships by using expensive randomized control trials. Further, the randomized control trials entail several logistical and ethical constraints. Further, the conventional methods fails to perform CI in a multiple treatment scenario and suffers due to selection bias. In most of the conventional methods, the multiple treatments scenario is interpreted as different dosage levels of a single treatment, or being one of several treatments. Hence a CI framework for multiple treatments without cofounding and selection bias is challenging.

Document FREDRIK D JOHANSSON ETAL: "Learning Representations for Counterfactual Inference" shows that learning representations that encourage similarity (balance) between the treated and control populations leads to better counterfactual inference.

Document PATRICK SCHWABETAL: "Perfect Match: A Simple Method for Learning Representations For Counterfactual Inference With Neural Networks" discloses learning representations for counterfactual inference based on the idea of augmenting samples within a minibatch with their propensity-matched nearest neighbours.

SUMMARY

Embodiments of the present disclosure present technological improvements as solutions to one or more of the above-mentioned technical problems recognized by the inventors in conventional systems. For example, in one embodiment, a method for matched and balanced causal inference for multiple treatments is provided. The method includes receiving a Causal Inference (CI) data of a plurality of subjects under test, wherein the CI data includes a factual treatment data, a factual response data, a plurality of attributes associated with each of the plurality of subjects under test. Further, the method includes computing a Propensity Score (PS) for each of the plurality of subjects under test for a treatment based on the CI data by using a predictive model, wherein the PS is a conditional probability of each of the subject under test, for responding to the treatment. Furthermore, the method includes computing a Generalized Propensity Score (GPS) for each of the plurality of subjects under test for a plurality of treatments based on the corresponding Propensity Score (PS). Furthermore, the method includes augmenting a plurality of task batches using the GPS, wherein each of the plurality of task batches comprises a plurality of sample subjects from the plurality of subjects under test, wherein augmenting each of the plurality of task batches comprising: for each sample subject $x_i$ from the plurality of sample subjects, with factual treatment $t_i$, a plurality of nearest neighbor sample subjects $x_j$ with observed treatment $t_j$ is selected based on the corresponding GPS. Furthermore, the method includes training a Deep Neural Network (DNN) using the plurality of augmented task batches to obtain balancing representation, wherein the DNN comprises a balancing network comprising a plurality of balancing branches and a hypothesis network comprising a plurality of hypothesis branches corresponding to the plurality of treatments, wherein steps of training the DNN comprising: (i) computing a balanced representation by training the balancing network until a difference between distribution of balancing network outputs from each of the plurality of balancing layers for a plurality of distinct treatments is minimum (ii) computing a factual response for each of the plurality of treatments from the corresponding branch of the hypothesis network based on the balancing representation (iii) computing a factual error by computing absolute difference between the factual response and an actual response and (iv) optimizing the DNN using a loss function based on the factual error and a balancing error, wherein the balancing error is a minimum mean discrepancy between pairwise distributions of two different treatments Finally, the method includes predicting a plurality of counter factual treatment response corresponding to the factual treatment data for each of the plurality of subjects under test using the trained DNN.

**EP 3 961 510 B1**

In another aspect, a system for matched and balanced causal inference for multiple treatments is provided. The system includes at least one memory storing programmed instructions, one or more Input /Output (I/O) interfaces, and one or more hardware processors operatively coupled to the at least one memory, wherein the one or more hardware processors are configured by the programmed instructions to receive a Causal Inference (CI) data of a plurality of subjects under test, wherein the CI data comprises a factual treatment data, a factual response data, a plurality of attributes associated with each of the plurality of subjects under test. Further, the one or more hardware processors are configured by the programmed instructions to compute a Propensity Score (PS) for each of the plurality of subjects under test for a treatment based on the CI data by using a predictive model, wherein the PS is a conditional probability of each of the subject under test, for responding to the treatment. Further, the one or more hardware processors are configured by the programmed instructions to compute a Generalized Propensity Score (GPS) for each of the plurality of subjects under test for a plurality of treatments based on the corresponding Propensity Score (PS). Furthermore, the one or more hardware processors are configured by the programmed instructions to augment a plurality of task batches using the GPS, wherein each of the plurality of task batches comprises a plurality of sample subjects from the plurality of subjects under test, wherein augmenting each of the plurality of task batches comprising: for each sample subject $x_i$ from the plurality of sample subjects, with factual treatment $t_i$, a plurality of nearest neighbor sample subjects $x_j$ with observed treatment $t_j$ is selected based on the corresponding GPS. Furthermore, the one or more hardware processors are configured by the programmed instructions to train a Deep Neural Network (DNN) using the plurality of augmented task batches to obtain balancing representation, wherein the DNN comprises a balancing network comprising a plurality of balancing branches and a hypothesis network comprising a plurality of hypothesis branches corresponding to the plurality of treatments, wherein steps of training the DNN comprising: (i) computing a balanced representation by training the balancing network until a difference between distribution of balancing network outputs from each of the plurality of balancing layers for a plurality of distinct treatments is minimum (ii) computing a factual response for each of the plurality of treatments from the corresponding branch of the hypothesis network based on the balancing representation (iii) computing a factual error by computing absolute difference between the factual response and an actual response and (iv) optimizing the DNN using a loss function based on the factual error and a balancing error, wherein the balancing error is a minimum mean discrepancy between pairwise distributions of two different treatments. Finally, the one or more hardware processors are configured by the programmed instructions to predict a plurality of counter factual treatment response corresponding to the factual treatment data for each of the plurality of subjects under test using the trained DNN.

In yet another aspect, a computer program product including a non-transitory computer-readable medium having embodied therein a computer program for method and system for matched and balanced causal inference for multiple treatments is provided. The computer readable program, when executed on a computing device, causes the computing device to receive a Causal Inference (CI) data of a plurality of subjects under test, wherein the CI data comprises a factual treatment data, a factual response data, a plurality of attributes associated with each of the plurality of subjects under test. Further, the computer readable program, when executed on a computing device, causes the computing device to compute a Propensity Score (PS) for each of the plurality of subjects under test for a treatment based on the CI data by using a predictive model, wherein the PS is a conditional probability of each of the subject under test, for responding to the treatment. Furthermore, the computer readable program, when executed on a computing device, causes the computing device to compute a Generalized Propensity Score (GPS) for each of the plurality of subjects under test for a plurality of treatments based on the corresponding Propensity Score (PS). Furthermore, the computer readable program, when executed on a computing device, causes the computing device to augment a plurality of task batches using the GPS, wherein each of the plurality of task batches comprises a plurality of sample subjects from the plurality of subjects under test, wherein augmenting each of the plurality of task batches comprising: for each sample subject $x_i$ from the plurality of sample subjects, with factual treatment $t_i$, a plurality of nearest neighbor sample subjects $x_j$ with observed treatment $t_j$ is selected based on the corresponding GPS. Furthermore, the computer readable program, when executed on a computing device, causes the computing device to train a Deep Neural Network (DNN) using the plurality of augmented task batches to obtain balancing representation, wherein the DNN comprises a balancing network comprising a plurality of balancing branches and a hypothesis network comprising a plurality of hypothesis branches corresponding to the plurality of treatments, wherein steps of training the DNN comprising: (i) computing a balanced representation by training the balancing network until a difference between distribution of balancing network outputs from each of the plurality of balancing layers for a plurality of distinct treatments is minimum (ii) computing a factual response for each of the plurality of treatments from the corresponding branch of the hypothesis network based on the balancing representation (iii) computing a factual error by computing absolute difference between the factual response and an actual response and (iv) optimizing the DNN using a loss function based on the factual error and a balancing error, wherein the balancing error is a minimum mean discrepancy between pairwise distributions of two different treatments. Finally, the computer readable program, when executed on a computing device, causes the computing device to predict a plurality of counter factual treatment response corresponding to the factual treatment data for each of the plurality of subjects under test using the trained DNN.

The invention is set out in the appended set of claims.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system for matched and balanced causal inference for multiple treatments, according to some embodiments of the present disclosure.

FIG. 2A and 2B are exemplary flow diagrams for a method for matched and balanced causal inference for multiple treatments implemented by the system of FIG. 1, in accordance with some embodiments of the present disclosure.

FIG. 3 illustrates an architectural overview of the system of FIG. 1 for matched and balanced causal inference for multiple treatments, in accordance with some embodiments of the present disclosure.

FIG. 4A to 5D illustrates experimental results of the system of FIG. 1 for matched and balanced causal inference for multiple treatments, in accordance with some embodiments of the present disclosure.

DETAILED DESCRIPTION OF EMBODIMENTS

Exemplary embodiments are described with reference to the accompanying drawings. In the figures, the leftmost digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following claims.

Embodiments herein provide a method and system for matched and balanced causal inference for multiple treatments to predict a counter factual treatment response corresponding to a factual treatment data for a plurality of subjects under test in an accurate manner. The system for matched and balanced causal inference for multiple treatments provides an accurate prediction of counter factual treatment response corresponding to a factual treatment data using a trained Deep Neural Network (DNN). Initially, a Causal Inference (CI) data pertaining to the plurality of subjects under test is given as input to the system. A Propensity Score (PS) is computed for the plurality of subjects under test for a treatment and a Generalized Propensity Score (GPS) is computed for a plurality of treatments by using the PS. Further, a plurality of task batches are created using the GPS and given as input to the DNN for training. The DNN includes a balancing network and a hypothesis network. The balancing network removes the selection bias and the balanced representation is given as input to the hypothesis network for computing factual data. An error in factual data and balancing representation is rectified using a novel loss function. The trained DNN is further used for predicting the counter factual treatment response corresponding to the factual treatment data for the plurality of subjects under test. The counter factual treatment is defined as, a probable effect of one or more different treatments for a subject, i.e., "what would have happened if the same subject is given a different treatment".

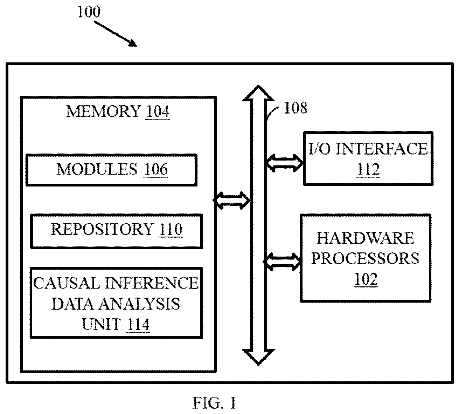

Referring now to the drawings, and more particularly to FIG. 1 through 5D, where similar reference characters denote corresponding features consistently throughout the figures, there are shown preferred embodiments and these embodiments are described in the context of the following exemplary system and/or method.

FIG. 1 is a functional block diagram of a system 100 for matched and balanced causal inference for multiple treatments, according to some embodiments of the present disclosure. The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the interface 112 may enable the system 100 to communicate with other devices, such as web servers and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting a number of computing systems with one another or to another server computer. The I/O interface 112 may include one or more ports for connecting a number of devices to one another or to another server.

The one or more hardware processors 102 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 104 includes a plurality of modules 106 and a causal inference data analysis unit 114. The memory 104 also includes a data repository 110 for storing data processed, received, and generated by the plurality of modules 106 and the causal inference data analysis unit 114.

The plurality of modules 106 include programs or coded instructions that supplement applications or functions performed by the system 100 for matched and balanced causal inference for multiple treatments. The plurality of modules 106, amongst other things, can include routines, programs, objects, components, and data structures, which performs particular tasks or implement particular abstract data types. The plurality of modules 106 may also be used as, signal processor(s), state machine(s), logic circuitries, and/or any other device or component that manipulates signals based on operational instructions. Further, the plurality of modules 106 can be used by hardware, by computer-readable instructions executed by a processing unit, or by a combination thereof. The plurality of modules 106 can include various sub-modules (not shown). The plurality of modules 106 may include computer-readable instructions that supplement applications or functions performed by the system 100 for matched and balanced causal inference for multiple treatments.

The data repository 110 may include a plurality of abstracted piece of code for refinement and data that is processed, received, or generated as a result of the execution of the plurality of modules in the module(s) 106 and the modules associated with the causal inference data analysis unit 114. The data repository may also include CI data, training and test data associated with the machine learning model used in the method for matched and balanced causal inference for multiple treatments.

Although the data repository 110 is shown internal to the system 100, it will be noted that, in alternate embodiments, the data repository 110 can also be implemented external to the system 100, where the data repository 110 may be stored within a database (not shown in FIG. 1) communicatively coupled to the system 100. The data contained within such external database may be periodically updated. For example, new data may be added into the database (not shown in FIG. 1) and/or existing data may be modified and/or non-useful data may be deleted from the database (not shown in FIG. 1). In one example, the data may be stored in an external system, such as a Lightweight Directory Access Protocol (LDAP) directory and a Relational Database Management System (RDBMS).

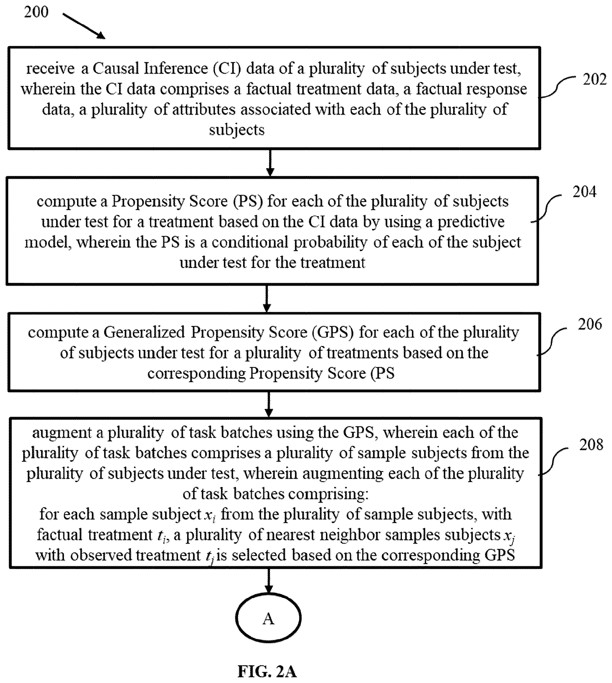

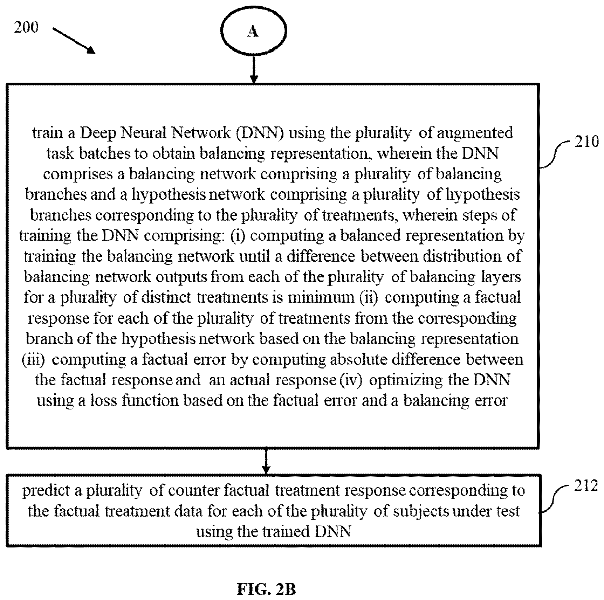

FIG. 2A and 2B are exemplary flow diagrams for a processor implemented method for matched and balanced causal inference for multiple treatments implemented by the system of FIG. 1, according to some embodiments of the present disclosure. In an embodiment, the system 100 comprises one or more data storage devices or the memory 104 operatively coupled to the one or more hardware processor(s) 102 and is configured to store instructions for execution of steps of the method 200 by the one or more hardware processors 102. The steps of the method 200 of the present disclosure will now be explained with reference to the components or blocks of the system 100 as depicted in FIG. 1 and the steps of flow diagram as depicted in FIG. 2A and FIG. 2B. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

At step 202 of the method 200, the one or more hardware processors (102) receive a Causal Inference (CI) data of a plurality of subjects under test. The CI data includes a factual treatment data, a factual response data, a plurality of attributes including height, weight, blood pressure associated with each of the plurality of subjects under test. The factual treatment data is a one hot vector and the factual response data is a continuous random vector.

At step 204 of the method 200, the one or more hardware processors (102) compute a Propensity Score (PS) for each of the plurality of subjects under test for a treatment based on the CI data by using a predictive model. The PS is a conditional probability of each of the subject under test for the treatment. The predictive model is a pre-trained classifier, wherein the pre-trained classifier can be one of a random forest, or Support Vector Machine (SVM).

At 206 of the method 200, the one or more hardware processors (102) compute a Generalized Propensity Score (GPS) for each of the plurality of subjects under test for a plurality of treatments based on the corresponding Propensity Score (PS). The plurality of treatment is interchangeably referred as multiple treatment.

At 208 of the method 200, the one or more hardware processors (102) augment a plurality of task batches using the GPS. Each of the plurality of task batches includes a plurality of sample subjects from the plurality of subjects under test. The method of augmenting each of the plurality of task batches is: for each sample subject $x_i$ from the plurality of sample subjects, with factual treatment $t_i$, a plurality of nearest neighbor sample subjects $x_j$ with observed treatment $t_j$ is selected based on the corresponding GPS.

At 210 of the method 200, the one or more hardware processors (102) train a Deep Neural Network (DNN) using the plurality of augmented task batches to obtain balancing representation. The DNN includes a balancing network with a plurality of balancing branches and a hypothesis network including a plurality of hypothesis branches corresponding to the plurality of treatments. The method of training the DNN is performed as explained below: (i) a balanced representation is computed initially by training the balancing network until a difference between distribution of balancing network outputs from each of the plurality of balancing layers for a plurality of distinct treatments is minimum (ii) a factual response is computed for each of the plurality of treatments from the corresponding branch of the hypothesis network based on the balancing representation (iii) a factual error is computed by computing absolute difference between the factual response and an actual response and (iv) the DNN is optimized by using a loss function based on the factual error and a balancing error. The loss function keeps the factual error less than a pre-determined factual threshold and the balancing error less than a pre-determined balancing threshold. The balancing error is a minimum mean discrepancy between pairwise distributions of two different treatments.

At 212 of the method 200, the one or more hardware processors (102) predict a plurality of counter factual treatment response corresponding to the factual treatment data for each of the plurality of subjects under test using the trained DNN.

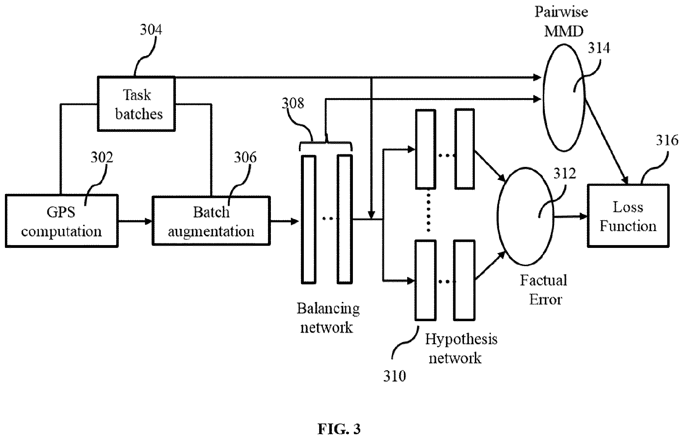

FIG. 3 illustrates an architectural overview of the system of FIG. 1 for matched and balanced causal inference for multiple treatments, in accordance with some embodiments of the present disclosure. Now referring to FIG. 3, the functional block diagram includes a GPS computation module 302, a task batches module 304, a batch augmentation module 306, a balancing network 308, a hypothesis network 310, a factual error computation module 312, a pairwise MMD (Minimum Mean Discrepancy) module 314 and a loss computation module 316. In an embodiment, the modules explained with FIG. 3 are present in the causal inference data analysis unit 114.

In an embodiment, the GPS computation module 302, computes the GPS score for the plurality of treatments for a subject by using the PS. PS is the conditional probability of a given individual $x_i$ receiving a treatment $t_k$, i.e., $p(t_k|x_i)$. Accordingly, the GPS vector is defined as $p(t|x_i) = [p(t_1|x_i),p(t_2|x_i),...,p(t_K|x_i)]$.

In an embodiment, the task batches module 304, creates the plurality of task batches. The plurality of task batches includes the plurality of sample subjects from the plurality of subjects under test. Each sample is further augmented using the batch augmentation module 306.

In an embodiment, the batch augmentation module 306, performs augmentation in the plurality of sample subjects from the plurality of subjects under test associated with each task batch. The batch augmentation is performed in such a way that, for each sample subject $x_i$ from the plurality of sample subjects, with factual treatment $t_i$, the plurality nearest neighbor sample subjects $x_j$ with observed treatment $t_j$ is selected based on the corresponding GPS. For example, if a subject S1 is given the factual treatment t1, a subject S2 is given the observed treatment t2, a subject S3 is given the observed treatment t3 and a subject S4 is given the observed treatment t3 for a disease D. Let the GPS for the subject S3 for the observed treatment t3 for the disease D is 80 and the GPS for the subject S4 for the observed treatment t3 for the disease D is 60. The subject S3 with the observed treatment t3 is selected to be included in the sample over the subject S4 with the observed treatment t3 since the GPS for the subject S3 with the observed treatment t3 is greater than the GPS for the subject S4 with the observed treatment t3.

In an embodiment, the balancing network 308, computes a balancing representation for the plurality of task batches by training the balancing network until the difference between distributions of balancing network outputs from each of the plurality of balancing layers for a plurality of distinct treatments is minimum. For example, for one of the cases the discrepancy metric for two treatments, '11' and 't2' was 0.30.

The hypothesis network 310 computes the factual response for each of the plurality of treatments from the corresponding branch of the hypothesis network based on the balancing representation. For example, if there are 3 treatments namely '11', 't2' and 't3', the hypothesis network is having 3 separate dedicated branches b1, b2 and b3 (one for each treatment) and all the samples having treatment '11' will pass through 'b1', treatment 't2' will pass through 'b2' and treatment 't3' will pass through 'b3'.

In an embodiment, the factual error computation module 312, computes the factual error by computing absolute difference between the predicted factual response and an actual response. For example, for a treatment '11' the corresponding actual response is 'y1' and predicted response for the same treatment '11' as 'py1', then the factual error would be the absolute value of 'py1 - y1'.

In an embodiment, the pairwise MMD module 314 computes a pairwise MMD for the plurality of treatments. For example, if there are total t1, t2 and t3 treatments an let output of balance network be phi(X), then it is the measure between phi(X) for subjects offered treatment '11' and treatment 't2' plus measure between phi(X) for subjects offered factual treatment 't2' and treatment 't3' plus phi(X) for subjects offered treatment 't3' and treatment 't1'.

In an embodiment, the loss computation module 316 optimizes the DNN using the loss function based on the factual error and the balancing error. The loss function keeps the factual error less than the pre-determined factual threshold and the balancing error less than the pre-determined balancing threshold. The values of the pre-determined factual threshold and the balancing error less than the pre-determined balancing threshold varies based on the data set used. The balancing error is The MMD between pairwise distributions of two different treatments as explained in the module 314.

The causal inference data analysis unit 114, executed by the one or more processors of the system 100, receives the Causal Inference (CI) data of the plurality of subjects under test. The CI data includes the factual treatment data, the factual response data and the plurality of attributes associated with each of the plurality of subjects under test. The factual treatment data is the one hot vector and the factual response data is the continuous random vector. For example, the CI data includes N samples, where each sample is given by $\{x_i, t, y_i\}$. Each subject under test i is represented using covariates given by $x_i$ for $1 \leq i \leq N$. The subject under test is subjected to one of the $K$ treatments given by t = $[t_1,...,t_K]$, where each entry of t is binary, i. e., $t_k \in \{0,1\}$. Here, $t_k = 1$ implies that the k-th treatment is given. It is assumed that only one treatment is provided to a subject i at any given point in time, and hence, t is a one-hot vector. Accordingly, the response vector for the i-th individual is given by $y_i \in R^{K \times 1}$, i.e., the outcome is a continuous random vector with $K$ entries denoted by $y_{ix}$, the response of the $i$-th individual to the $k$-th treatment. The counterfactual treatment is defined as the $K$-1 alternate treatments which are unobserved for the subject under test.

Further, the causal inference data analysis unit 114, executed by one or more processors of the system 100, computes the Propensity Score (PS) for each of the plurality of subjects under test for the treatment based on the CI data by using the predictive model, wherein the PS is a conditional probability of each of the subject under test for the treatment. The predictive model is the pre-trained classifier, wherein the pre-trained classifier includes one of the random forest or the Support Vector Machine (SVM).

In an embodiment, the predictive model is trained as follows: The dataset $D_{PS}$ is divided into train, validation and test sets. The predictive model (SVM or Random Forest) is trained over the train set and its parameters are tuned using the validation set. Furthermore, the tuned model is used to make predictions over the test set (which is the data for CI)

Further, the causal inference data analysis unit 114, executed by one or more processors of the system 100, computes the Generalized Propensity Score (GPS) for each of the plurality of subjects under test for the plurality of treatments based on the corresponding Propensity Score (PS). PS is the conditional probability of a subject under test $x_i$ receiving a treatment $t_k$, i.e., $p(t_k|x_i)$. Accordingly, the GPS vector is defined as $p(\mathbf{t}|x_i) = [p(t_1|x_i),p(t_2|x_i),...,p(t_K|x_i)]$. In order to avoid overfitting, $l$ nearest neighbors are selected and one out of these $l$ samples is picked at random for each counterfactual treatment of $x_i$.

Further, the causal inference data analysis unit 114, executed by one or more processors of the system 100, augments the plurality of task batches using the GPS. Each of the plurality of task batches includes the plurality of sample subjects from the plurality of subjects under test, wherein augmenting each of the plurality of task batches is performed as follows: for each sample subject $x_i$ from the plurality of sample subjects, with factual treatment $t_i$, the plurality of nearest neighbor sample subjects $x_j$ with observed treatment $t_j$ is selected based on the corresponding GPS.

In an embodiment, the GPS vector $p(\mathbf{t}|x_i)$ is applied for batch augmentation in every task batch. For every sample within a task batch, $K - 1$ closest neighbor samples are obtained. For instance, consider a sample $x_i$ and its factual treatment $t_i$. The GPS-based matching $M_{GPS}$ is applied to select a neighbor $x_j$ with observed treatment $t_j$ such that $t_j \neq t_i$ and $d_{GPS}(i,j)$ is minimum. Here, $d_{GPSi,j}$ is defined in equation 1.

$$d_{GPS}(i,j) = \sum_{k=1}^{K} |p(t_k|x_i) - p(t_k|x_j)| \dots\dots\dots\dots\dots\dots (1)$$

Further, the causal inference data analysis unit 114, executed by one or more processors of the system 100, trains the Deep Neural Network (DNN) using the plurality of augmented task batches to obtain balancing representation. The DNN includes the balancing network with a plurality of balancing branches and the hypothesis network including the plurality of hypothesis branches corresponding to the plurality of treatments. The method of training the DNN is performed as explained below: (i) the balanced representation is computed initially by training the balancing network until a difference between distribution of balancing network outputs from each of the plurality of balancing layers for a plurality of distinct treatments is minimum (ii) the factual response is computed for each of the plurality of treatments from the corresponding branch of the hypothesis network based on the balancing representation (iii) the factual error is computed by computing absolute difference between the factual response and an actual response and (iv) the DNN is optimized by using a loss function based on the factual error and a balancing error. The loss function keeps the factual error less than the pre-determined factual threshold and the balancing error less than the pre-determined balancing threshold. The balancing error is the MMD between pairwise distributions of two different treatments.

In an embodiment, the loss function is given in equation 2.

$$\mathcal{L}(\alpha, \gamma) = \frac{1}{N} \sum_{i=1}^{N} \sum_{k=1}^{K} |(h(\Phi(x_i), t_k) - y_{ik})| + \alpha \sum_{m=1}^{K} \sum_{q=1}^{m-1} \text{disc}(\hat{p}_{\Phi}^{m}, \hat{p}_{\Phi}^{q}) + \gamma \mathcal{R}(h)$$

$$\dots\dots\dots\dots\dots\dots\dots (2)$$

where $\alpha, \gamma > 0$ are hyperparameters controlling the strength of the imbalance penalties, R(h) is a model complexity term, $\hat{p}^m(\cdot)$ and $\hat{p}(^q\cdot)$ represent the distribution corresponding to the $m$-th treatment and the $q$-th treatment, respectively, and disc$(\cdot, \cdot)$ is the MMD measure. The balancing representation $\Phi(\cdot)$ and the hypothesis $h(\cdot)$ are learnt jointly by training the DNN using the loss function given in equation 2 that incorporates the factual and the imbalance error. In the equation 2, the first term on the right hand side represents the factual loss. The second term computes the pairwise MMD between factual distributions of different treatments. The loss function in equation 2 is a generalization to the multiple treatment scenario.

In an embodiment, the procedure for performing the present disclosure is give below:

1: procedure MultiMBNN( D )

2:     Split dataset $D$ into $D_{CI}$ for CI, and $D_{PS}$ to compute GPS.

3:     Divide $D_{CI}$ into train ($D_{CI,t}$), validation and test sets.

4:     Obtain GPS $p(\text{t}|x_i), \forall i$ in $D_{CI,t}$,

5:     Divide $D_{CI,t}$ into batches with each batch being $M_B$

6:     for $E$ epochs and $M_B \in D_{CI,t}$ do

7:         $\tilde{M}_B \leftarrow$ augment $M_B$ using $M_{GPS}$,

8:         Update $\Phi(\cdot)$, $h(\cdot)$ using input $\tilde{M}_B$ by minimizing

        equation 2

        return $\Phi(\cdot)$, $h(\cdot)$

Further, the causal inference data analysis unit 114, executed by one or more processors of the system 100, predicts the plurality of counter factual treatment response corresponding to the factual treatment data for each of the plurality of subjects under test using the trained DNN. For example, if there are two possible treatments, '11' and 't2' for a subject S1, where '11' is the factual treatment, then the predicted counterfactual treatment response for the subject S1 is y1' = 10.24 for '11' and 'y2' = 20.12 for 't2'.

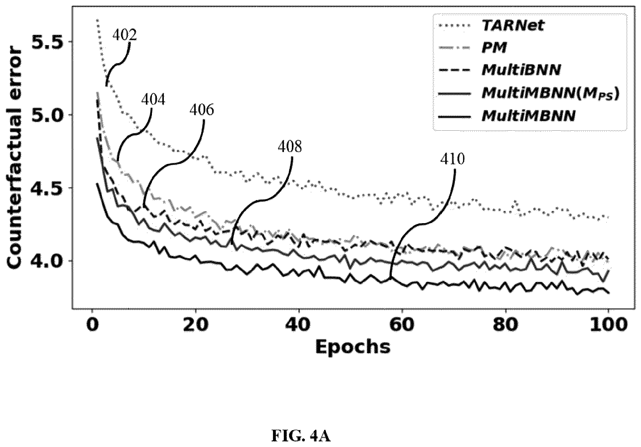

In an embodiment, the present disclosure is experimented as follows: FIG. 4A to 5D illustrates experimental results of the system of FIG. 1 for matched and balanced causal inference for multiple treatments, in accordance with some embodiments of the present disclosure.

The performance of MultiMBNN (Multi Matched and Balanced Neural Network) algorithm is tested on a plurality of data sets including synthetic, semisynthetic NEWS and cancer genome TCGA (The Cancer Cell Genome Atlas) datasets. In an embodiment, 15000 samples including 10 covariates for the synthetic dataset with $\kappa$ accounting for the treatment assignment bias is used for experimentation. A DGP (Data generation Process) approach is utilized and 5772 bag-of-words context covariates are used to generate N = 10000 samples of the NEWS dataset. In the case of synthetic and NEWS datasets, the generate data for $K$ = 4,6,8 are generated and referred to as 'name of the dataset', followed by $K$. The TCGA dataset consisting of 10000 samples with 20547 covariates is obtained using the DGP with $K$ = 4 ('TCGA4'). The PEHE (Precision in Estimation of Heterogeneous treatment Effect) (denoted as $\varepsilon_P$) and

$$ATE = \frac{1}{N} \sum_{i=1}^{N} |y_{ik} - \frac{1}{K-1} \sum_{j=1, t_j \neq t_k}^{K-1} y_{ij}|$$ are used for measuring the performance.

The present disclosure is tested using TARNet (Treatment Agnostic Representation Netwoek) and the performance of the present disclosure (MultiMBNN algorithm) using several experimental settings. First, the effect of treatment assignment bias using the parameter $\kappa$ is illustrated. Here, SYN (Synthetic dataset) 4 is tested for 4 values of $\kappa$ and NEWS4 for 3 values of $\kappa$, respectively.

FIG. 4A to FIG. 4C illustrates a comparison of CI frameworks based on counter factual errors across epochs

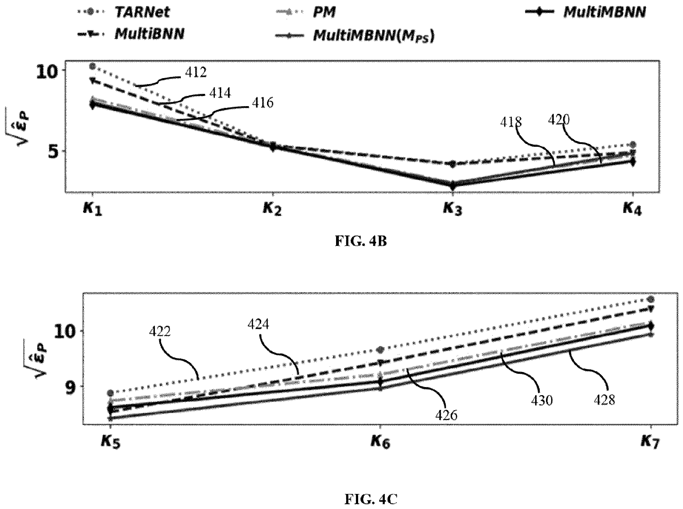

Syn4 vs $\kappa_1$ and $\sqrt{\hat{\epsilon}_P}$ vs. $\kappa$. Here FIG. 4A illustrates the graph plotted between Syn4 vs $\kappa_1$. Now referring to FIG. 4A, plot 402, 404, 406, 408 and 410 indicates the performance of the present disclosure using TARNet, PM (Perfect Match), MultiBNN, MultiMBNN (Mps) (MultiMBNN with PS Matching) and MultiMBNN respectively. FIG. 4B illustrates the graph plotted between Syn4 with $\kappa_1 < \kappa_2 < \kappa_3 < \kappa_4$. Now referring to FIG. 4B, the plots 412, 414, 416, 418 and 420 illustrates the performance of the present disclosure for TARNet, PM, MultiBNN, MultiMBNN (Mps) and MultiMBNN respectively. FIG. 4C illustrates the graph plotted between NEWS4 with $\kappa_5 < \kappa_6 < \kappa_7$. Now referring to FIG. 4C, plots 422, 424, 426, 428 and 430 illustrates the performance of the present disclosure for TARNet, PM, MultiBNN, MultiMBNN (Mps) and MultiMBNN respectively It is observed that the MultiMBNN algorithm performs better for $\kappa_3$ of SYN4 since imbalance amongst treatments leads to one of the four treatments to be suppressed which lead to a large counterfactual error and hence, an elbow point. For NEWS4, $\kappa_5$ has the least counterfactual error since imbalance amongst treatment groups is minimum, leading to near uniform distribution of population samples in all four groups.

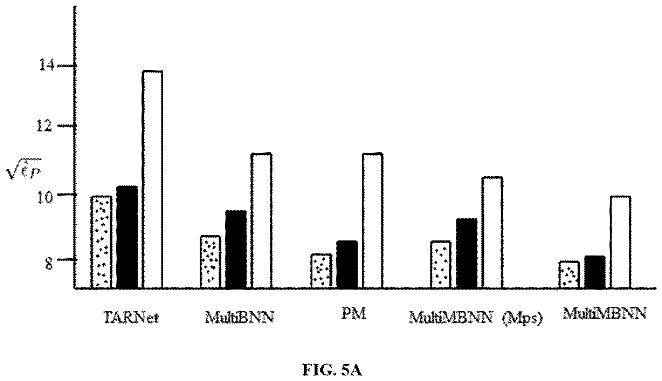

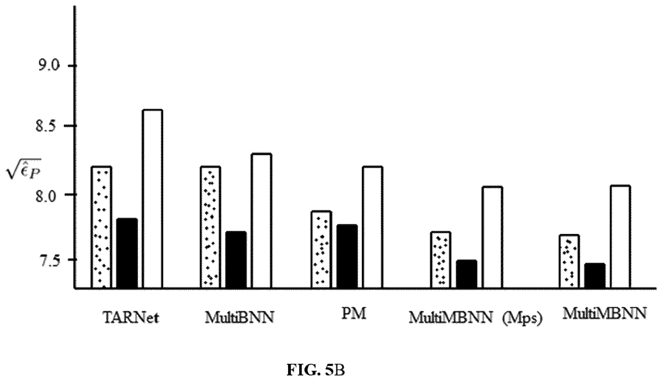

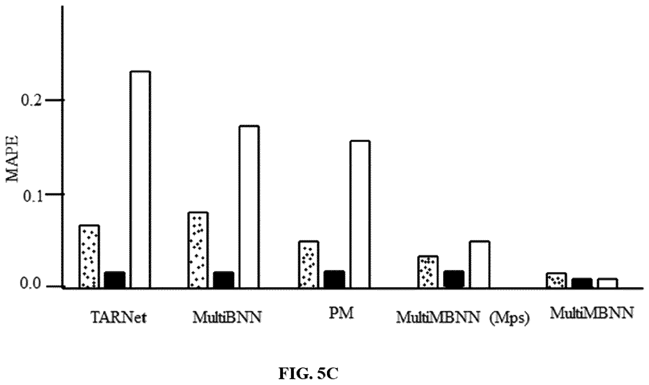

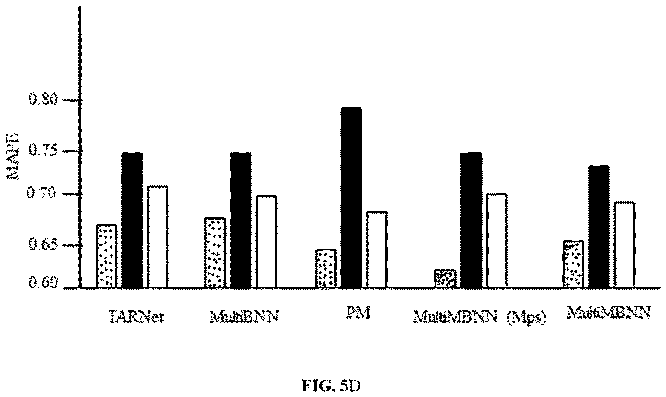

FIG. 5A to 5D illustrates the performance of the present disclosure with varying $K$ for a fixed $\kappa$. FIG. 5A and FIG. 5B illustrates the bar diagrams plotted for various frameworks with varying values of $\sqrt{\hat{\epsilon}_P}$. FIG. 5C and FIG. 5D illustrates the bar diagram plotted between MAPE (Mean Absolute Percentage Error) of various frameworks. Now referring to FIGs 5A to 5D, the dotted bar indicates the SYN4 dataset, the solid black bar is for SYN6 dataset and white bar is for SYN8 dataset. Referring to FIGs. 5A to 5D, the MultiMBNN algorithm outperforms the baselines by large margins. Further, the MultiMBNN with different initial seed-points maintaining $K$ and $\kappa$ fixed, is simulated, and the mean and standard deviation in $\sqrt{\hat{\epsilon}_P}$ and *MAPE* for all baselines is reported in Table 1. It is inferred that the MultiMBNN which incorporates both matching and DNN based balancing, fairs considerably well over all the baselines.

Table 1

| Metrics,Dataset | TARNet | MultiBNN | PM | MultiMBNN($M_{PS}$) | MultiMBNN |
|---|---|---|---|---|---|
| $\sqrt{\hat{\epsilon}_P}$, Syn4 | 10.21 ± 0.56 | 9.34 ± 0.61 | 8.21 ± 0.35 | 7.98 ± 0.23 | 7.86 ± 0.37 |
| MAPE, Syn4 | 0.07 ± 0.02 | 0.08 ± 0.03 | 0.06 ± 0.02 | 0.04 ± 0.01 | 0.02 ± 0.02 |
| $\sqrt{\hat{\epsilon}_P}$, NEWS4 | 9.70 ± 1.15 | 9.37 ± 0.90 | 9.16 ± 0.80 | 8.99 ± 0.94 | 8.96 ± 0.92 |
| MAPE, NEWS4 | 0.81 ± 0.11 | 0.81 ± 0.10 | 0.81 ± 0.12 | 0.80±0.13 | 0.82 ± 0.12 |
| $\sqrt{\hat{\epsilon}_P}$, TCGA4 | 29.45 ± 3.48 | 26.15 ± 3.29 | 23.57 ± 1.10 | 23.18 ± 1.39 | 21.47 ± 0.96 |
| MAPE, TCGA4 | 0.93 ± 0.14 | 0.84 ± 0.10 | 0.92 ± 0.07 | 0.78 ± 0.03 | 0.80 0.08 |

The written description describes the subject matter herein to enable any person skilled in the art to make and use the embodiments. The scope of the subject matter embodiments is defined by the claims.

The embodiments of present disclosure herein address unresolved problem of addressing inadequacies of the matching framework by learning the balanced representations in multiple treatment causal inference scenario. Here, the multiple treatment scenario is achieved by using the GPS technique. Further, the accuracy of the system 100 is achieved by using the DNN by employing the loss function given in equation 2. The loss function eliminates the factual error and the balancing error. The present disclosure can be extended for handling sparsity in the presence of large number of treatments.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs, GPUs and edge computing devices.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer readable medium can be any apparatus that can comprise, store, communicate, propagate, or transport the program for use by or in connection with the instruction execution system, apparatus, or device. The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise. Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e. non-transitory. Examples include random access memory (RAM), read-only memory (ROM), volatile memory, nonvolatile memory, hard drives, CD ROMs, DVDs, flash drives, disks, and any other known physical storage media.

## Claims

1.   A processor implemented method (200), the method comprising:

    receiving, by one or more hardware processors (102), a Causal Inference (CI) data of a plurality of subjects under test, wherein the CI data comprises a factual treatment data, a factual response data, a plurality of attributes associated with each of the plurality of subjects under test (202);
    computing, by the one or more hardware processors (102), a Propensity Score (PS) for each of the plurality of subjects under test for a treatment based on the CI data by using a predictive model, wherein the PS is a conditional probability of each of the subject under test, for responding to the treatment (204);
    computing, by the one or more hardware processors (102), a Generalized Propensity Score (GPS) for each of the plurality of subjects under test for a plurality of treatments based on the corresponding Propensity Score (PS) (206);
    augmenting, by the one or more hardware processors (102), a plurality of task batches using the GPS, wherein each of the plurality of task batches comprises a plurality of sample subjects from the plurality of subjects under test, wherein augmenting each of the plurality of task batches (208) comprising:
    for each sample subject $x_i$ from the plurality of sample subjects with factual treatment $t_i$, a plurality of nearest neighbor sample subjects $x_j$ with observed treatment $t_j$ is selected based on the corresponding GPS, wherein the GPS is a vector describing a conditional probability of the each sample subject $x_i$, receiving a treatment $t_k$, wherein the GPS vector is defined as $p(t|x_i) = [p(t_1|x_i), p(t_2|x_i), ..., p(t_K|x_i)]$, wherein the GPS vector is applied for augmenting the plurality of task batches and a GPS based matching is applied to select the plurality of nearest neighbor sample subjects $x_j$ with the observed treatment $t_j$ such that tj ≠ ti and $d_{GPS}$ (i,j) is minimum, wherein the $d_{GPS}$ (i,j) is defined by an equation

$$d_{GPS}(i, j) = \sum_{k=1}^{K} |p(t_k|x_i) - p(t_k|x_j)|$$ ;

    training, by the one or more hardware processors (102), a Deep Neural Network (DNN) using the augmented plurality of task batches to obtain balancing representation, wherein the DNN comprises a balancing network comprising a plurality of balancing branches whose output is given as input to a a hypothesis network comprising a plurality of hypothesis branches corresponding to the plurality of treatments, wherein steps of training the DNN (210) comprising:

        computing a balanced representation by training the balancing network until a difference between distribution of outputs from each layer of the balancing network for a plurality of distinct treatments is minimum;

computing a factual response for each of the plurality of treatments from the corresponding branch of the hypothesis network based on the balancing representation;

computing a factual error by computing absolute difference between the factual response and an actual response; and

optimizing the DNN using a loss function based on the factual error and a balancing error; and

predicting, by the one or more hardware processors (102), a plurality of counter factual treatment response corresponding to the factual treatment data for each of the plurality of subjects under test using the trained DNN (212).

2. The method (200) of claim 1, wherein the predictive model is a pre-trained classifier, wherein the pre-trained classifier comprises one of a random forest, and a Support Vector Machine (SVM).

3. The method (200) of claim 1, wherein the loss function controls the factual error less than a pre-determined factual threshold and the balancing error less than a pre-determined balancing threshold.

4. The method (200) of claim 1, wherein the factual treatment data is a one hot vector and the factual response data is a continuous random vector.

5. A system (100) comprising:

at least one memory (104) storing programmed instructions;
one or more Input /Output (I/O) interfaces (112); and
one or more hardware processors (102) operatively coupled to the at least one memory (104), wherein the one or more hardware processors (102) are configured by the programmed instructions to:

receive a Causal Inference (CI) data of a plurality of subjects under test, wherein the CI data comprises a factual treatment data, a factual response data, a plurality of attributes associated with each of the plurality of subjects under test;

compute a Propensity Score (PS) for each of the plurality of subjects under test for a treatment based on the CI data by using a predictive model, wherein the PS is a conditional probability of each of the subject under test, for responding to the treatment;

compute a Generalized Propensity Score (GPS) for each of the plurality of subjects under test for a plurality of treatments based on the corresponding Propensity Score (PS);

augment a plurality of task batches using the GPS, wherein each of the plurality of task batches comprises a plurality of sample subjects from the plurality of subjects under test, wherein augmenting each of the plurality of task batches comprising:

for each sample subject $x_i$ from the plurality of sample subjects with factual treatment $t_i$, a plurality of nearest neighbor sample subjects $x_j$ with observed treatment $t_j$ is selected based on the corresponding GPS, wherein the GPS is a vector describing a conditional probability of the each sample subject $x_i$, receiving a treatment $t_k$, wherein the GPS vector is defined as $p(t|x_i) = [p(t_1|x_i), p(t_2|x_i),..., p(t_K|x_i)]$, wherein the GPS vector is applied for augmenting the plurality of task batches and a GPS based matching is applied to select the plurality of nearest neighbor sample subjects $x_j$ with the observed treatment $t_j$ such that tj t-ti and $d_{GPS}$ (i,j) is minimum, wherein the $d_{GPS}$ (i,j) is defined by an equation

$$d_{GPS}(i,j) = \sum_{k=1}^{K} |p(t_k|x_i) - p(t_k|x_j)|;$$

train a Deep Neural Network (DNN) using the augmented plurality of task batches to obtain balancing representation, wherein the DNN comprises a balancing network comprising a plurality of balancing branches whose output is given as input to a a hypothesis network comprising a plurality of hypothesis branches corresponding to the plurality of treatments, wherein steps of training the DNN comprising:

computing a balanced representation by training the balancing network until a difference between distribution of outputs from each layer of the balancing network for a plurality of distinct treatments is minimum;

computing a factual response for each of the plurality of treatments from the corresponding branch of the hypothesis network based on the balancing representation;

computing a factual error by computing absolute difference between the factual response and an actual response; and

optimizing the DNN using a loss function based on the factual error and a balancing error, wherein the balancing error is a minimum mean discrepancy between pairwise distributions of two different treatments; and

predict a plurality of counter factual treatment response corresponding to the factual treatment data for each of the plurality of subjects under test using the trained DNN.

6. The system (100) of claim 5, the predictive model is a pre-trained classifier, wherein the pre-trained classifier comprises one of a random forest, and a Support Vector Machine (SVM).

7. The system (100) of claim 5, wherein the loss function controls the factual error less than a pre-determined factual threshold and the balancing error less than a pre-determined balancing threshold.

8. The system (100) of claim 5, wherein the factual treatment data is a one hot vector and the factual response data is a continuous random vector.

9. One or more non-transitory machine readable information storage mediums comprising one or more instructions which when executed by one or more hardware processors (102) causes:

receiving a Causal Inference (CI) data of a plurality of subjects under test, wherein the CI data comprises a factual treatment data, a factual response data, a plurality of attributes associated with each of the plurality of subjects under test (202);

computing Propensity Score (PS) for each of the plurality of subjects under test for a treatment based on the CI data by using a predictive model, wherein the PS is a conditional probability of each of the subject under test, for responding to the treatment (204);

computing a Generalized Propensity Score (GPS) for each of the plurality of subjects under test for a plurality of treatments based on the corresponding Propensity Score (PS) (206);

augmenting a plurality of task batches using the GPS, wherein each of the plurality of task batches comprises a plurality of sample subjects from the plurality of subjects under test, wherein augmenting each of the plurality of task batches (208) comprising:

for each sample subject $x_i$ from the plurality of sample subjects with factual treatment $t_i$, a plurality of nearest neighbor sample subjects $x_j$ with observed treatment $t_j$ is selected based on the corresponding GPS, wherein the GPS is a vector describing a conditional probability of the each sample subject $x_i$, receiving a treatment $t_k$, wherein the GPS vector is defined as $p(t|x_i) = [p(t_1|x_i),p(t_2|x_i),...,p(t_K|x_i)]$, wherein the GPS vector is applied for augmenting the plurality of task batches and a GPS based matching is applied to select the plurality of nearest neighbor sample subjects $x_j$ with the observed treatment $t_j$ such that tj ≠ ti and $d_{GPS}$ (i,j) is minimum, wherein the $d_{GPS}$ (i,j) is defined by an equation

$$d_{GPS}(i,j) = \sum_{k=1}^{K} |p(t_k|x_i) - p(t_k|x_j)|,$$

training a Deep Neural Network (DNN) using the augmented plurality of task batches to obtain balancing representation, wherein the DNN comprises a balancing network comprising a plurality of balancing branches whose output is given as input to a a hypothesis network comprising a plurality of hypothesis branches corresponding to the plurality of treatments, wherein steps of training the DNN (210) comprising:

computing a balanced representation by training the balancing network until a difference between distribution of outputs from each layer of the balancing network for a plurality of distinct treatments is minimum;

computing a factual response for each of the plurality of treatments from the corresponding branch of the hypothesis network based on the balancing representation;

computing a factual error by computing absolute difference between the factual response and an actual response; and

optimizing the DNN using a loss function based on the factual error and a balancing error; and

predicting a plurality of counter factual treatment response corresponding to the factual treatment data for each of the plurality of subjects under test using the trained DNN (212).

10. The one or more non-transitory machine readable information storage mediums of claim 9, wherein the loss function controls the factual error less than a pre-determined factual threshold and the balancing error less than a pre-determined balancing threshold and , wherein the factual treatment data is a one hot vector and the factual response data is a continuous random vector.

11. The one or more non-transitory machine readable information storage mediums of claim 9, wherein the loss function controls the factual error less than a pre-determined factual threshold and the balancing error less than a pre-determined balancing threshold.

12. The one or more non-transitory machine readable information storage mediums of claim 9, wherein the factual treatment data is a one hot vector and the factual response data is a continuous random vector.

**Patentansprüche**

1. Prozessorimplementiertes Verfahren (200), wobei das Verfahren Folgendes umfasst:

Empfangen, durch einen oder mehrere Hardwareprozessoren (102), von kausalen Inferenzdaten (CI-Daten) einer Mehrzahl von zu testenden Subjekten, wobei die CI-Daten faktische Behandlungsdaten, faktische Antwortdaten und eine Mehrzahl von Attributen, die jedem der Mehrzahl von zu testenden Subjekten (202) zugeordnet sind, umfassen;
Berechnen, durch den einen oder die mehreren Hardwareprozessoren (102), einer Neigungsbewertung (PS) für jedes der Mehrzahl von zu testenden Subjekten für eine Behandlung basierend auf den CI-Daten durch Verwenden eines prädiktiven Modells, wobei die PS eine bedingte Wahrscheinlichkeit jedes der zu testenden Subjekte ist, auf die Behandlung zu antworten (204);
Berechnen, durch den einen oder die mehreren Hardwareprozessoren (102), einer generalisierten Neigungsbewertung (GPS) für jedes der Mehrzahl von zu testenden Subjekten für eine Mehrzahl von Behandlungen basierend auf der entsprechenden Neigungsbewertung (PS) (206);
Erweitern, durch den einen oder die mehreren Hardwareprozessoren (102), einer Mehrzahl von Aufgabenstapeln unter Verwendung des GPS, wobei jeder der Mehrzahl von Aufgabenstapeln eine Mehrzahl von Probensubjekten aus der Mehrzahl von zu testenden Subjekten umfasst, wobei das Erweitern jedes der Mehrzahl von Aufgabenstapeln (208) Folgendes umfasst:
für jedes Probensubjekt $x_i$ aus der Mehrzahl von Probensubjekten mit faktischer Behandlung $t_i$ wird eine Mehrzahl von nächstliegenden Probensubjekten $x_j$ mit beobachteter Behandlung $t_j$ basierend auf dem entsprechenden GPS ausgewählt, wobei das GPS ein Vektor ist, der eine bedingte Wahrscheinlichkeit jedes Probensubjekts $x_i$ beschreibt, Empfangen einer Behandlung $t_k$, wobei der GPS-Vektor als $p(t|x_i) = [p(t_1|x_i),p(t_2|x_i),...,p(t_K|x_i)]$ definiert ist, wobei der GPS-Vektor zum Erweitern der Mehrzahl von Aufgabenstapeln angewendet wird und ein GPS-basierter Abgleich angewendet wird, um die Mehrzahl von nächstliegenden Probensubjekten $x_j$ mit der beobachteten Behandlung $t_j$ so auszuwählen, dass tj t- ti und $d_{GPS}$ (i,j) minimal ist, wobei das $d_{GPS}$ (i,j) durch

$$d_{GPS}(i,j) = \sum_{k=1}^{K} |p(t_k|x_i) - p(t_k|x_j)|$$

eine Gleichung                              definiert ist;
Trainieren, durch den einen oder die mehreren Hardwareprozessoren (102), eines tiefen neuronalen Netzwerks (DNN) unter Verwendung der erweiterten Mehrzahl von Aufgabenstapeln, um eine ausgleichende Darstellung zu erhalten, wobei das DNN ein ausgleichendes Netzwerk umfasst, das eine Mehrzahl von ausgleichenden Zweigen umfasst, deren Ausgabe als Eingabe in ein Hypothesennetzwerk gegeben ist, das eine Mehrzahl von Hypothesenzweigen umfasst, die der Mehrzahl von Behandlungen entsprechen, wobei die Schritte des Trainierens des DNN (210) Folgendes umfassen:

Berechnen einer ausgeglichenen Darstellung durch Trainieren des ausgleichenden Netzwerks, bis eine Differenz zwischen der Verteilung von Ausgaben aus jeder Schicht des ausgleichenden Netzwerks für eine Mehrzahl von unterschiedlichen Behandlungen minimal ist;
Berechnen einer faktischen Antwort für jede der Mehrzahl von Behandlungen aus dem entsprechenden Zweig des Hypothesennetzwerks basierend auf der ausgleichenden Darstellung;
Berechnen eines faktischen Fehlers durch Berechnen einer absoluten Differenz zwischen der faktischen Antwort und einer tatsächlichen Antwort; und
Optimieren des DNN unter Verwendung einer Verlustfunktion basierend auf dem faktischen Fehler und einem ausgleichenden Fehler; und

Vorhersagen, durch den einen oder die mehreren Hardwareprozessoren (102), einer Mehrzahl von faktischen Behandlungsantwortzählern, die den faktischen Behandlungsdaten für jedes der Mehrzahl von zu testenden Subjekten entsprechen, unter Verwendung des trainierten DNN (212).

2. Verfahren (200) nach Anspruch 1, wobei das prädiktive Modell ein vortrainierter Klassifizierer ist, wobei der vortrainierte Klassifizierer eines von einem Zufallswald und einer Support Vector Machine (SVM) umfasst.

3. Verfahren (200) nach Anspruch 1, wobei die Verlustfunktion den faktischen Fehler, der kleiner als ein vorbestimmter faktischer Schwellenwert ist, und den ausgleichenden Fehler, der kleiner als ein vorbestimmter ausgleichender Schwellenwert ist, steuert.

4. Verfahren (200) nach Anspruch 1, wobei die faktischen Behandlungsdaten ein "one hot"-Vektor sind und die faktischen Antwortdaten ein kontinuierlicher Zufallsvektor sind.

5. System (100), das Folgendes umfasst:

mindestens einen Speicher (104), der programmierte Anweisungen speichert;
eine oder mehrere Eingabe/Ausgabe(E/A)-Schnittstellen (112); und
einen oder mehrere Hardwareprozessoren (102), die betriebsfähig mit dem mindestens einen Speicher (104) gekoppelt sind, wobei der eine oder die mehreren Hardwareprozessoren (102) durch die programmierten Anweisungen konfiguriert sind zum:

Empfangen von kausalen Inferenzdaten (CI-Daten) einer Mehrzahl von zu testenden Subjekten, wobei die CI-Daten faktische Behandlungsdaten, faktische Antwortdaten und eine Mehrzahl von Attributen, die jedem der Mehrzahl von zu testenden Subjekten zugeordnet sind, umfassen;
Berechnen einer Neigungsbewertung (PS) für jedes der Mehrzahl von zu testenden Subjekten für eine Behandlung basierend auf den CI-Daten durch Verwenden eines prädiktiven Modells, wobei die PS eine bedingte Wahrscheinlichkeit jedes der zu testenden Subjekte ist, auf die Behandlung zu antworten;
Berechnen einer generalisierten Neigungsbewertung (GPS) für jedes der Mehrzahl von zu testenden Subjekten für eine Mehrzahl von Behandlungen basierend auf der entsprechenden Neigungsbewertung (PS);
Erweitern einer Mehrzahl von Aufgabenstapeln unter Verwendung des GPS, wobei jeder der Mehrzahl von Aufgabenstapeln eine Mehrzahl von Probensubjekten aus der Mehrzahl von zu testenden Subjekten umfasst, wobei das Erweitern jedes der Mehrzahl von Aufgabenstapeln Folgendes umfasst:
für jedes Probensubjekt $x_i$ aus der Mehrzahl von Probensubjekten mit faktischer Behandlung $t_i$ wird eine Mehrzahl von nächstliegenden Probensubjekten $x_j$ mit beobachteter Behandlung $t_j$ basierend auf dem entsprechenden GPS ausgewählt, wobei das GPS ein Vektor ist, der eine bedingte Wahrscheinlichkeit jedes Probensubjekts $x_i$ beschreibt, Empfangen einer Behandlung $t_k$, wobei der GPS-Vektor als $p(t|x_i) = [p(t_1|x_i),p(t_2|x_i),...,p(t_K|x_i)]$ definiert ist, wobei der GPS-Vektor zum Erweitern der Mehrzahl von Aufgabenstapeln angewendet wird und ein GPS-basierter Abgleich angewendet wird, um die Mehrzahl von nächstliegenden Probensubjekten $x_j$ mit der beobachteten Behandlung $t_j$ so auszuwählen, dass tj ≠ ti und d$_{GPS}$(i,j) minimal ist, wobei das d$_{GPS}$ (i,j) durch eine Gleichung

$$d_{GPS}(i,j) = \sum_{k=1}^{K} |p(t_k|x_i) - p(t_k|x_j)|$$

definiert ist;
Trainieren eines tiefen neuronalen Netzwerks (DNN) unter Verwendung der erweiterten Mehrzahl von Aufgabenstapeln, um eine ausgleichende Darstellung zu erhalten, wobei das DNN ein ausgleichendes Netzwerk umfasst, das eine Mehrzahl von ausgleichenden Zweigen umfasst, deren Ausgabe als Eingabe in ein Hypothesennetzwerk gegeben ist, das eine Mehrzahl von Hypothesenzweigen umfasst, die der Mehrzahl von Behandlungen entsprechen, wobei die Schritte des Trainierens des DNN Folgendes umfassen:

Berechnen einer ausgeglichenen Darstellung durch Trainieren des ausgleichenden Netzwerks, bis eine Differenz zwischen der Verteilung von Ausgaben aus jeder Schicht des ausgleichenden Netzwerks für eine Mehrzahl von unterschiedlichen Behandlungen minimal ist;
Berechnen einer faktischen Antwort für jede der Mehrzahl von Behandlungen aus dem entsprechenden Zweig des Hypothesennetzwerks basierend auf der ausgleichenden Darstellung;

Berechnen eines faktischen Fehlers durch Berechnen einer absoluten Differenz zwischen der faktischen Antwort und einer tatsächlichen Antwort; und

Optimieren des DNN unter Verwendung einer Verlustfunktion basierend auf dem faktischen Fehler und einem ausgleichenden Fehler, wobei der ausgleichende Fehler eine minimale mittlere Diskrepanz zwischen paarweisen Verteilungen von zwei verschiedenen Behandlungen ist; und

Vorhersagen einer Mehrzahl von faktischen Behandlungsantwortzählern, die den faktischen Behandlungsdaten für jedes der Mehrzahl von zu testenden Subjekten entsprechen, unter Verwendung des trainierten DNN.

6. System (100) nach Anspruch 5, wobei das prädiktive Modell ein vortrainierter Klassifizierer ist, wobei der vortrainierte Klassifizierer eines von einem Zufallswald und einer Support Vector Machine (SVM) umfasst.

7. System (100) nach Anspruch 5, wobei die Verlustfunktion den faktischen Fehler, der kleiner als ein vorbestimmter faktischer Schwellenwert ist, und den ausgleichenden Fehler, der kleiner als ein vorbestimmter ausgleichender Schwellenwert ist, steuert.

8. System (100) nach Anspruch 5, wobei die faktischen Behandlungsdaten ein "one hot"-Vektor sind und die faktischen Antwortdaten ein kontinuierlicher Zufallsvektor sind.

9. Ein oder mehrere nichtflüchtige maschinenlesbare Informationsspeichermedien, die eine oder mehrere Anweisungen umfassen, die, wenn sie durch einen oder mehrere Hardwareprozessoren (102) ausgeführt werden, Folgendes bewirken:

Empfangen von kausalen Inferenzdaten (CI-Daten) einer Mehrzahl von zu testenden Subjekten, wobei die CI-Daten faktische Behandlungsdaten, faktische Antwortdaten und eine Mehrzahl von Attributen, die jedem der Mehrzahl von zu testenden Subjekten (202) zugeordnet sind, umfassen;

Berechnen einer Neigungsbewertung (PS) für jedes der Mehrzahl von zu testenden Subjekten für eine Behandlung basierend auf den CI-Daten durch Verwenden eines prädiktiven Modells, wobei die PS eine bedingte Wahrscheinlichkeit jedes der zu testenden Subjekte ist, auf die Behandlung zu antworten (204);

Berechnen einer generalisierten Neigungsbewertung (GPS) für jedes der Mehrzahl von zu testenden Subjekten für eine Mehrzahl von Behandlungen basierend auf der entsprechenden Neigungsbewertung (PS) (206);

Erweitern einer Mehrzahl von Aufgabenstapeln unter Verwendung des GPS, wobei jeder der Mehrzahl von Aufgabenstapeln eine Mehrzahl von Probensubjekten aus der Mehrzahl von zu testenden Subjekten umfasst, wobei das Erweitern jedes der Mehrzahl von Aufgabenstapeln (208) Folgendes umfasst:

für jedes Probensubjekt $x_i$ aus der Mehrzahl von Probensubjekten mit faktischer Behandlung $t_i$ wird eine Mehrzahl von nächstliegenden Probensubjekten $x_j$ mit beobachteter Behandlung $t_j$ basierend auf dem entsprechenden GPS ausgewählt, wobei das GPS ein Vektor ist, der eine bedingte Wahrscheinlichkeit jedes Probensubjekts $x_i$ beschreibt, Empfangen einer Behandlung $t_k$, wobei der GPS-Vektor als $p(t|x_i) = [p(t_1|x_i), p(t_2|x_i), ..., p(t_K|x_i)]$ definiert ist, wobei der GPS-Vektor zum Erweitern der Mehrzahl von Aufgabenstapeln angewendet wird und ein GPS-basierter Abgleich angewendet wird, um die Mehrzahl von nächstliegenden Probensubjekten $x_j$ mit der beobachteten Behandlung $t_j$ so auszuwählen, dass tj t- ti und $d_{GPS}(i,j)$ minimal ist, wobei das $d_{GPS}(i,j)$ durch eine Gleichung $d_{GPS}(i,j) = \sum_{k=1}^{K} |p(t_k|x_i) - p(t_k|x_j)|$ definiert ist;

Trainieren eines tiefen neuronalen Netzwerks (DNN) unter Verwendung der erweiterten Mehrzahl von Aufgabenstapeln, um eine ausgleichende Darstellung zu erhalten, wobei das DNN ein ausgleichendes Netzwerk umfasst, das eine Mehrzahl von ausgleichenden Zweigen umfasst, deren Ausgabe als Eingabe in ein Hypothesennetzwerk gegeben ist, das eine Mehrzahl von Hypothesenzweigen umfasst, die der Mehrzahl von Behandlungen entsprechen, wobei die Schritte des Trainierens des DNN (210) Folgendes umfassen:

Berechnen einer ausgeglichenen Darstellung durch Trainieren des ausgleichenden Netzwerks, bis eine Differenz zwischen der Verteilung von Ausgaben aus jeder Schicht des ausgleichenden Netzwerks für eine Mehrzahl von unterschiedlichen Behandlungen minimal ist;

Berechnen einer faktischen Antwort für jede der Mehrzahl von Behandlungen aus dem entsprechenden Zweig des Hypothesennetzwerks basierend auf der ausgleichenden Darstellung;

Berechnen eines faktischen Fehlers durch Berechnen einer absoluten Differenz zwischen der faktischen Antwort und einer tatsächlichen Antwort; und

Optimieren des DNN unter Verwendung einer Verlustfunktion basierend auf dem faktischen Fehler und einem ausgleichenden Fehler; und

Vorhersagen einer Mehrzahl von faktischen Behandlungsantwortzählern, die den faktischen Behandlungsdaten für jedes der Mehrzahl von zu testenden Subjekten entsprechen, unter Verwendung des trainierten DNN (212).

10. Ein oder mehrere nichtflüchtige maschinenlesbare Informationsspeichermedien nach Anspruch 9, wobei die Verlustfunktion den faktischen Fehler, der kleiner als ein vorbestimmter faktischer Schwellenwert ist, und den ausgleichenden Fehler, der kleiner als ein vorbestimmter ausgleichender Schwellenwert ist, steuert, und wobei die faktischen Behandlungsdaten ein "one hot"-Vektor sind und die faktischen Antwortdaten ein kontinuierlicher Zufallsvektor sind.

11. Ein oder mehrere nichtflüchtige maschinenlesbare Informationsspeichermedien nach Anspruch 9, wobei die Verlustfunktion den faktischen Fehler, der kleiner als ein vorbestimmter faktischer Schwellenwert ist, und den ausgleichenden Fehler, der kleiner als ein vorbestimmter ausgleichender Schwellenwert ist, steuert.

12. Ein oder mehrere nichtflüchtige maschinenlesbare Informationsspeichermedien nach Anspruch 9, wobei die faktischen Behandlungsdaten ein "one hot"-Vektor sind und die faktischen Antwortdaten ein kontinuierlicher Zufallsvektor sind.

## Revendications

1. Procédé mis en oeuvre par processeur (200), le procédé comprenant les étapes ci-dessous consistant à :

recevoir, par le biais d'un ou plusieurs processeurs matériels (102), des données d'inférence causale (CI) d'une pluralité de sujets testés, dans lesquelles les données d'inférence CI comprennent des données de traitement factuel, des données de réponse factuelle et une pluralité d'attributs associés à chaque sujet de la pluralité de sujets testés (202) ;

calculer, par le biais dudit un ou desdits plusieurs processeurs matériels (102), un score de propension (PS) pour chaque sujet de la pluralité de sujets testés, pour un traitement, sur la base des données d'inférence CI, à l'aide d'un modèle prédictif, dans lequel le score PS est une probabilité conditionnelle que chaque sujet des sujets testés réponde au traitement (204) ;

calculer, par le biais dudit un ou desdits plusieurs processeurs matériels (102), un score de propension généralisé (GPS) pour chaque sujet de la pluralité de sujets testés, pour une pluralité de traitements, sur la base du score de propension (PS) correspondant (206) ;

augmenter, par le biais dudit un ou desdits plusieurs processeurs matériels (102), une pluralité de lots de tâches, à l'aide du score GPS, dans lequel chaque lot de la pluralité de lots de tâches comprend une pluralité de sujets échantillons provenant de la pluralité de sujets testés, dans lequel l'étape d'augmentation de chaque lot de la pluralité de lots de tâches (208) comprend l'étape ci-dessous dans laquelle :

pour chaque sujet échantillon $x_i$ de la pluralité de sujets échantillons avec un traitement factuel $t_i$, une pluralité de sujets échantillons voisins les plus proches $x_j$ avec un traitement observé $t_j$ est sélectionnée sur la base du score GPS correspondant, dans lequel le score GPS est un vecteur décrivant une probabilité conditionnelle que ledit chaque sujet échantillon $x_i$ reçoive un traitement $t_k$, dans lequel le vecteur de score GPS est défini en tant que $p(t|x_i) = [p(t_1|x_i), p(t_2|x_i), ..., p(t_{K|}x_i)]$, dans lequel le vecteur de score GPS est appliqué pour augmenter la pluralité de lots de tâches, et une mise en correspondance à base de score GPS est appliquée pour sélectionner la pluralité de sujets échantillons voisins les plus proches $x_j$ avec le traitement observé $t_j$, de sorte que $t_j \neq t_i$ et $d_{GPS}$ (i, j) est minimum, dans lequel $d_{GPS}$ (i,j) est défini par une équation

$$d_{GPS}(i,j) = \sum_{k=1}^{K} |p(t_k|x_i) - p(t_k|x_j)| ;$$

entraîner, par le biais dudit un ou desdits plusieurs processeurs matériels (102), un réseau de neurones profonds (DNN), en utilisant la pluralité augmentée de lots de tâches, en vue d'obtenir une représentation d'équilibrage, dans lequel le réseau DNN comprend un réseau d'équilibrage comprenant une pluralité de branches d'équilibrage dont la sortie est donnée en entrée à un réseau d'hypothèse comprenant une pluralité de branches d'hypothèse correspondant à la pluralité de traitements, dans lequel les étapes d'entraînement du réseau DNN (210) comprennent les étapes ci-dessous consistant à :
calculer une représentation équilibrée en entraînant le réseau d'équilibrage jusqu'à ce qu'une différence entre une distribution de sorties provenant de chaque couche du réseau d'équilibrage pour une pluralité de traitements distincts soit minimale :

  calculer une réponse factuelle, pour chaque traitement de la pluralité de traitements, à partir de la branche correspondante du réseau d'hypothèse, sur la base de la représentation d'équilibrage ;
  calculer une erreur factuelle en calculant une différence absolue entre la réponse factuelle et une réponse réelle ; et
  optimiser le réseau DNN, à l'aide d'une fonction de perte, sur la base de l'erreur factuelle et d'une erreur d'équilibrage ; et

  prédire, par le biais dudit un ou desdits plusieurs processeurs matériels (102), une pluralité de réponses au traitement contre-factuel correspondant aux données de traitement factuel pour chaque sujet de la pluralité de sujets testés, à l'aide du réseau DNN entraîné (212).

2. Procédé (200) selon la revendication 1, dans lequel le modèle prédictif est un classificateur pré-entraîné, dans lequel le classificateur pré-entraîné comprend l'un des éléments parmi une forêt aléatoire et une machine à vecteurs de support (SVM).

3. Procédé (200) selon la revendication 1, dans lequel la fonction de perte commande l'erreur factuelle inférieure à un seuil factuel prédéterminé et l'erreur d'équilibrage inférieure à un seuil d'équilibrage prédéterminé.

4. Procédé (200) selon la revendication 1, dans lequel les données de traitement factuel sont un vecteur d'encodage « 1 parmi n » et les données de réponse factuelle sont un vecteur aléatoire continu.

5. Système (100), comprenant :

  au moins une mémoire (104) stockant des instructions programmées ;
  une ou plusieurs interfaces d'entrée/sortie (I/O) (112) ; et
  un ou plusieurs processeurs matériels (102) couplés fonctionnellement à ladite au moins une mémoire (104), dans lesquels ledit un ou lesdits plusieurs processeurs matériels (102) sont configurés, par les instructions programmées, de manière à mettre en oeuvre les étapes ci-dessous consistant à :

    recevoir des données d'inférence causale (CI) d'une pluralité de sujets testés, dans lesquelles les données d'inférence CI comprennent des données de traitement factuel, des données de réponse factuelle et une pluralité d'attributs associés à chaque sujet de la pluralité de sujets testés ;
    calculer un score de propension (PS) pour chaque sujet de la pluralité de sujets testés, pour un traitement, sur la base des données d'inférence CI, à l'aide d'un modèle prédictif, dans lequel le score PS est une probabilité conditionnelle que chaque sujet des sujets testés réponde au traitement ;
    calculer un score de propension généralisé (GPS) pour chaque sujet de la pluralité de sujets testés, pour une pluralité de traitements, sur la base du score de propension (PS) correspondant ;
    augmenter une pluralité de lots de tâches, à l'aide du score GPS, dans lequel chaque lot de la pluralité de lots de tâches comprend une pluralité de sujets échantillons provenant de la pluralité de sujets testés, dans lequel l'étape d'augmentation de chaque lot de la pluralité de lots de tâches comprend l'étape ci-dessous dans laquelle :
    pour chaque sujet échantillon $x_i$ de la pluralité de sujets échantillons avec un traitement factuel $t_i$, une pluralité de sujets échantillons voisins les plus proches $x_j$ avec un traitement observé $t_j$ est sélectionnée sur la base du score GPS correspondant, dans lequel le score GPS est un vecteur décrivant une probabilité conditionnelle que ledit chaque sujet échantillon $x_i$ reçoive un traitement $t_k$, dans lequel le vecteur de score GPS est défini en tant que $p(t|x_i) = [p(t_1|x_i), p(t_2|x_i), ..., p(t_K|x_i)]$, dans lequel le vecteur de score GPS est appliqué pour augmenter la pluralité de lots de tâches, et une mise en correspondance à base de score GPS est appliquée pour sélectionner la pluralité de sujets échantillons voisins les plus proches $x_j$ avec le traitement observé $t_j$, de sorte que $t_j \neq t_i$ et $d_{GPS}(i, j)$ est minimum, dans lequel $d_{GPS}(i,j)$ est défini par une

équation $d_{GPS}(i,j) = \sum_{k=1}^{K} |p(t_k|x_i) - p(t_k|x_j)|$ ;

entraîner un réseau de neurones profonds (DNN), en utilisant la pluralité augmentée de lots de tâches, en vue d'obtenir une représentation d'équilibrage, dans lequel le réseau DNN comprend un réseau d'équilibrage comprenant une pluralité de branches d'équilibrage dont la sortie est donnée en entrée à un réseau d'hypothèse comprenant une pluralité de branches d'hypothèse correspondant à la pluralité de traitements, dans lequel les étapes d'entraînement du réseau DNN comprennent les étapes ci-dessous consistant à : calculer une représentation équilibrée en entraînant le réseau d'équilibrage jusqu'à ce qu'une différence entre une distribution de sorties provenant de chaque couche du réseau d'équilibrage pour une pluralité de traitements distincts soit minimale :

> calculer une réponse factuelle, pour chaque traitement de la pluralité de traitements, à partir de la branche correspondante du réseau d'hypothèse, sur la base de la représentation d'équilibrage ;
> calculer une erreur factuelle en calculant une différence absolue entre la réponse factuelle et une réponse réelle ; et
> optimiser le réseau DNN, à l'aide d'une fonction de perte, sur la base de l'erreur factuelle et d'une erreur d'équilibrage, dans laquelle l'erreur d'équilibrage est une divergence moyenne minimale entre des distributions par paire de deux traitements différents ; et

> prédire une pluralité de réponses au traitement contre-factuel correspondant aux données de traitement factuel pour chaque sujet de la pluralité de sujets testés, à l'aide du réseau DNN entraîné.

6. Système (100) selon la revendication 5, dans lequel le modèle prédictif est un classificateur pré-entraîné, dans lequel le classificateur pré-entraîné comprend l'un des éléments parmi une forêt aléatoire et une machine à vecteurs de support (SVM).

7. Système (100) selon la revendication 5, dans lequel la fonction de perte commande l'erreur factuelle inférieure à un seuil factuel prédéterminé et l'erreur d'équilibrage inférieure à un seuil d'équilibrage prédéterminé.

8. Système (100) selon la revendication 5, dans lequel les données de traitement factuel sont un vecteur d'encodage « 1 parmi n » et les données de réponse factuelle sont un vecteur aléatoire continu.

9. Un ou plusieurs supports non transitoires de stockage d'informations lisibles par machine comprenant une ou plusieurs instructions qui, lorsqu'elles sont exécutées par un ou plusieurs processeurs matériels (102), occasionnent la mise en oeuvre des étapes ci-dessous consistant à :

> recevoir des données d'inférence causale (CI) d'une pluralité de sujets testés, dans lesquelles les données d'inférence CI comprennent des données de traitement factuel, des données de réponse factuelle et une pluralité d'attributs associés à chaque sujet de la pluralité de sujets testés (202) ;
> calculer un score de propension (PS) pour chaque sujet de la pluralité de sujets testés, pour un traitement, sur la base des données d'inférence CI, à l'aide d'un modèle prédictif, dans lequel le score PS est une probabilité conditionnelle que chaque sujet des sujets testés réponde au traitement (204) ;
> calculer un score de propension généralisé (GPS) pour chaque sujet de la pluralité de sujets testés, pour une pluralité de traitements, sur la base du score de propension (PS) correspondant (206) ;
> augmenter une pluralité de lots de tâches, à l'aide du score GPS, dans lequel chaque lot de la pluralité de lots de tâches comprend une pluralité de sujets échantillons provenant de la pluralité de sujets testés, dans lequel l'étape d'augmentation de chaque lot de la pluralité de lots de tâches (208) comprend l'étape ci-dessous dans laquelle :
> pour chaque sujet échantillon $x_i$ de la pluralité de sujets échantillons avec un traitement factuel $t_i$, une pluralité de sujets échantillons voisins les plus proches $x_j$ avec un traitement observé $t_j$ est sélectionnée sur la base du score GPS correspondant, dans lequel le score GPS est un vecteur décrivant une probabilité conditionnelle que ledit chaque sujet échantillon $x_i$ reçoive un traitement $t_k$, dans lequel le vecteur de score GPS est défini en tant que $p(t|x_i) = [p(t_1|x_i), p(t_2|x_i), ..., p(t_K|x_i)]$, dans lequel le vecteur de score GPS est appliqué pour augmenter la pluralité de lots de tâches, et une mise en correspondance à base de score GPS est appliquée pour sélectionner la pluralité de sujets échantillons voisins les plus proches $x_j$ avec le traitement observé $t_j$, de sorte que $t_i \neq t_j$ et $d_{GPS}(i, j)$ est minimum, dans lequel $d_{GPS}(i,j)$ est défini par une équation

$$d_{GPS}(i,j) = \sum_{k=1}^{K} |p(t_k|x_i) - p(t_k|x_j)| \; ;$$

entraîner un réseau de neurones profonds (DNN), en utilisant la pluralité augmentée de lots de tâches, en vue d'obtenir une représentation d'équilibrage, dans lequel le réseau DNN comprend un réseau d'équilibrage comprenant une pluralité de branches d'équilibrage dont la sortie est donnée en entrée à un réseau d'hypothèse comprenant une pluralité de branches d'hypothèse correspondant à la pluralité de traitements, dans lequel les étapes d'entraînement du réseau DNN (210) comprennent les étapes ci-dessous consistant à :
calculer une représentation équilibrée en entraînant le réseau d'équilibrage jusqu'à ce qu'une différence entre une distribution de sorties provenant de chaque couche du réseau d'équilibrage pour une pluralité de traitements distincts soit minimale :

calculer une réponse factuelle, pour chaque traitement de la pluralité de traitements, à partir de la branche correspondante du réseau d'hypothèse, sur la base de la représentation d'équilibrage ;
calculer une erreur factuelle en calculant une différence absolue entre la réponse factuelle et une réponse réelle ; et
optimiser le réseau DNN, à l'aide d'une fonction de perte, sur la base de l'erreur factuelle et d'une erreur d'équilibrage ; et

prédire une pluralité de réponses au traitement contre-factuel correspondant aux données de traitement factuel pour chaque sujet de la pluralité de sujets testés, à l'aide du réseau DNN entraîné (212).

**10.** Ledit un ou lesdits plusieurs supports non transitoires de stockage d'informations lisibles par machine selon la revendication 9, dans lesquels la fonction de perte commande l'erreur factuelle inférieure à un seuil factuel prédéterminé et l'erreur d'équilibrage inférieure à un seuil d'équilibrage prédéterminé, et dans lesquels les données de traitement factuel sont un vecteur d'encodage « 1 parmi n » et les données de réponse factuelle sont un vecteur aléatoire continu.

**11.** Ledit un ou lesdits plusieurs supports non transitoires de stockage d'informations lisibles par machine selon la revendication 9, dans lesquels la fonction de perte commande l'erreur factuelle inférieure à un seuil factuel prédéterminé et l'erreur d'équilibrage inférieure à un seuil d'équilibrage prédéterminé.

**12.** Ledit un ou lesdits plusieurs supports non transitoires de stockage d'informations lisibles par machine selon la revendication 9, les données de traitement factuel sont un vecteur d'encodage « 1 parmi n » et les données de réponse factuelle sont un vecteur aléatoire continu.

100

MEMORY 104

MODULES 106

REPOSITORY 110

CAUSAL INFERENCE
DATA ANALYSIS
UNIT 114

108

I/O INTERFACE
112

HARDWARE
PROCESSORS
102

FIG. 1

200

receive a Causal Inference (CI) data of a plurality of subjects under test, wherein the CI data comprises a factual treatment data, a factual response data, a plurality of attributes associated with each of the plurality of subjects

202

compute a Propensity Score (PS) for each of the plurality of subjects under test for a treatment based on the CI data by using a predictive model, wherein the PS is a conditional probability of each of the subject under test for the treatment

204

compute a Generalized Propensity Score (GPS) for each of the plurality of subjects under test for a plurality of treatments based on the corresponding Propensity Score (PS

206

augment a plurality of task batches using the GPS, wherein each of the plurality of task batches comprises a plurality of sample subjects from the plurality of subjects under test, wherein augmenting each of the plurality of task batches comprising:
for each sample subject $x_i$ from the plurality of sample subjects, with factual treatment $t_i$, a plurality of nearest neighbor samples subjects $x_j$ with observed treatment $t_j$ is selected based on the corresponding GPS

208

A

**FIG. 2A**

200

A

train a Deep Neural Network (DNN) using the plurality of augmented
task batches to obtain balancing representation, wherein the DNN
comprises a balancing network comprising a plurality of balancing
branches and a hypothesis network comprising a plurality of hypothesis
branches corresponding to the plurality of treatments, wherein steps of
training the DNN comprising: (i) computing a balanced representation by
training the balancing network until a difference between distribution of
balancing network outputs from each of the plurality of balancing layers
for a plurality of distinct treatments is minimum (ii) computing a factual
response for each of the plurality of treatments from the corresponding
branch of the hypothesis network based on the balancing representation
(iii) computing a factual error by computing absolute difference between
the factual response and an actual response (iv) optimizing the DNN
using a loss function based on the factual error and a balancing error

210

predict a plurality of counter factual treatment response corresponding to
the factual treatment data for each of the plurality of subjects under test
using the trained DNN

212

**FIG. 2B**

FIG. 3

FIG. 4A

FIG. 4B

FIG. 4C

**FIG. 5A**

**FIG. 5B**

**FIG. 5C**

**FIG. 5D**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202021016253

**Non-patent literature cited in the description**

- **FREDRIK D JOHANSSON et al.** *Learning Representations for Counterfactual Inference*

- **PATRICK SCHWABETAL.** *Perfect Match: A Simple Method for Learning Representations For Counterfactual Inference With Neural Networks*